Europäisches Patentamt

**European Patent Office**

Office européen des brevets

(19)

(11) Publication number: **0 192 287**
**A2**

(12) # EUROPEAN PATENT APPLICATION

(21) Application number: **86200110.4**

(22) Date of filing: **27.01.86**

(51) Int. Cl.⁴: **C 07 D 213/61**
**C 07 D 213/73**

(30) Priority: **19.02.85 GB 8504268**

(43) Date of publication of application:
**27.08.86 Bulletin 86/35**

(84) Designated Contracting States:
**BE CH DE FR GB IT LI NL SE**

(71) Applicant: **SHELL INTERNATIONALE RESEARCH**
**MAATSCHAPPIJ B.V.**
**Carel van Bylandtlaan 30**
**NL-2596 HR Den Haag(NL)**

(72) Inventor: **Grotenhuis, Paulus Alexander Maria**
**Badhuisweg 3**
**NL-1031 CM Amsterdam(NL)**

(72) Inventor: **Prillwitz, Peter Ernst**
**Badhuisweg 3**
**NL-1031 CM Amsterdam(NL)**

(74) Representative: **Aalbers, Onno et al,**
**P.O. Box 302**
**NL-2501 CH The Hague(NL)**

(54) **Process for the preparation of fluorinated pyridines.**

(57) Process for the preparation of fluorinated pyridines in
which a chlorine-containing pyridine derivative is contacted
at elevated temperature with an alkali-metal fluoride in a
solvent wherein the solvent comprises a mixture of at least
one aprotic amide and at least one aromatic hydrocarbon.

EP 0 192 287 A2

K 589 FF

## PROCESS FOR THE PREPARATION OF FLUORINATED PYRIDINES

The invention relates to a process for the preparation of fluorinated pyridines.

It is known from the Journal of the Chemical Society, 1965, 594-597, to contact pentachloropyridine with potassium fluoride in an aprotic solvent, viz. N-methyl-2-pyrrolidone at 200°C under reflux, to yield fluorinated pyridine derivatives. Up to 74% of fluorinated pyridine derivatives were obtained, viz. up to 65% of 3,5-dichloro-2,4,6-trifluoropyridine and up to 9% of 3-chloro-2,4,5,6-tetrafluoropyridine. In the process according to the prior art the reaction is carried out in an amount of more than 870 grams of N-methylpyrrolidone per mol potassium fluoride. This amount of solvent is required in order to obtain a stirrable mixture.

It has now been found that the presence of an aromatic hydrocarbon compound has a tremendous impact on the selectivity and the yield of fluorine-containing pyridine derivatives. It appears that by changing the nature of the solvent higher yields of fluorine-containing pyridine derivatives can be obtained.

The present invention therefore provides a process for the preparation of a fluorinated pyridine in which a chlorine-containing pyridine derivative is contacted at elevated temperature with an alkali-metal fluoride in a solvent, wherein the solvent comprises a mixture of at least one aprotic amide and at least one aromatic hydrocarbon.

BK35.010

The process is especially applicable to the replacement of chlorine by fluorine in chlorine-containing pyridine derivatives which contain a chlorine substituent in the 2-, 4- and/or 6-position. The present process can be applied to pyridine derivatives which contain only one chlorine substituent, e.g. 2-chloro-3-nitro-pyridine or 4-chloro-3-cyano-pyridine. Preferably the pyridine derivative contains two or more chlorine substituents. A very suitable starting material for the process according to the invention is pentachloropyridine. When the latter compound is used in the process according to the invention not only a high yield of fluoropyridines is obtained, but also a high selectivity towards 3,5-dichloro-2,4,6-trifluoropyridine.

In the process according to the invention the alkali-metal fluoride used may conveniently be sodium, potassium, rubidium or cesium fluoride, potassium fluoride being particularly preferred. The amount of the alkali-metal fluoride is conveniently at least one mol of alkali-metal fluoride per gram atom of chlorine to be replaced. It is preferred to use an excess of alkali-metal fluoride. Especially when more than one chlorine substituent is present and not all chlorine substituents are to be replaced, for example as in the case of the preparation of 3,5-dichloro-2,4,6-trifluoropyridine from pentachloropyridine, the molar ratio of alkali-metal fluoride to chlorine atoms to be replaced is advantageously in the range 1:1 and 1.3:1.

The aprotic amide present in the solvent mixture may be for example an optionally substituted dialkylamide of a carboxylic acid containing up to 10 carbon atoms (e.g a dialkylformamide or a dialkylacetamide) or a N-alkyl-lactam (e.g. a N-alkyl-$\gamma$ -butyrolactam (a N-alkyl-pyrrolidone) or N-alkyl-caprolactam). The alkyl groups attached to nitrogen preferably contain from 1 to 6 carbon atoms. It is preferred to use N-methylpyrrolidone or N,N-dimethylacetamide.

Suitable aromatic hydrocarbons include benzene and alkyl- and alkenylbenzenes in which at least one $C_1-C_4$ alkyl or $C_{2-4}$ alkenyl group is connected to the benzene ring. Preferred are benzene, toluene, o-, m- or p-xylene or mixtures thereof, especially toluene.

Preferably, the weight ratio of aprotic amide to aromatic hydrocarbon is in the range from 10:90 to 90:10, more preferably from 65:35 to 20:80.

Use of the solvent comprising a mixture of at least one aprotic amide and at least one aromatic hydrocarbon generally allows a readily stirrable mixture to be obtained using slightly more than 150 grams of the solvent per mol of alkali-metal fluoride. Therefore the use of relatively small-scale equipment will suffice for the process according to the present invention. Of course it is possible to employ greater quantities of the solvent. The solvent is preferably present in an amount from 170-750 grams per mol of alkali-metal fluoride.

It is preferred to effect the process at a temperature in the range from 80°C to the reflux temperature of the reaction mixture. The reflux temperatures of the preferred solvent mixtures, e.g. mixtures of toluene and N-methylpyrrolidone or dimethyl acetamide within the preferred weight ratio range, are well below 200°C at atmospheric pressure, due to the boiling point decreasing effect of the toluene in the mixture. The possibility to operate in the present process at temperatures below 200°C represents a considerable economic advantage over the prior art process.

The present process may if desired be carried out at elevated pressures, e.g. up to 10 bar. This implies, that the reflux temperature of the solvent mixture increases. Reaction temperatures in the range 90 and 170°C, are preferred.

The process of the invention may be performed without a catalyst. However, it has been found that the reaction rate can be enhanced by using a phase transfer catalyst, and in the present process a phase transfer catalyst is preferably included in the reaction mixture. Suitable catalysts include crown ethers, e.g. 18-crown-6-ether, and quaternary ammonium and phosphonium compounds, e.g. tetramethylphosphoniumchloride. Preferably the phase transfer catalyst is a tetraalkyl ammonium halide, conveniently a tetraalkyl ammonium chloride. Each alkyl moiety in such a catalyst may conveniently be a $C_{1-6}$ alkyl group, $C_{1-4}$ alkyl groups, e.g. methyl and/or butyl groups, being preferred.

The presence of water in the reaction mixture is undesirable. The use of anhydrous reactants is therefore preferred. However, since alkali-metal fluorides are hygroscopic the presence of a little amount of water is almost unavoidable. Due to the use of an aromatic hydrocarbon-containing solvent mixture the reaction mixture can conveniently be dried by azeotropic distillation. Thereto the alkali-metal fluoride and the chlorine-containing pyridine derivative are preferably dried before admixture with the amide(s) by mixing them with the aromatic hydrocarbon(s) and removing water from the mixture obtained by azeotropic distillation. The distillate contains water and part of the aromatic hydrocarbon(s). The latter part can be dried in any known manner and be reused in the process according to the present invention.

A particularly preferred process in accordance with the present invention is a process for the preparation of 3,5-dichloro-2,4,6-trifluoropyridine in which pentachloropyridine is contacted with potassium fluoride, the molar ratio of potassium fluoride to pentachloropyridine being from 3:1 to 3.9:1, in a solvent wherein the solvent comprises a mixture of at least one aprotic amide selected from N-methylpyrrolidone, N,N-dimethylacetamide and N,N-dimethylformamide, and at least one aromatic hydrocarbon selected from benzene, toluene and o-, m- and p-xylene, the weight ratio between the aprotic amide(s) and the aromatic hydrocarbon(s) ranging from 65:35 to 20:80, and wherein the reaction temperature ranges from 80°C to the reflux temperature of the reaction mixture.

3,5-Dichloro-2,4,6-trifluoropyridine may conveniently be converted into the 4-amino-pyridine derivative by treatment with ammonia.

Accordingly, the invention also provides a process in accordance with the invention as defined above wherein the fluorinated pyridine is 4-amino-3,5-dichloro-2,6-difluoropyridine, in which pentachloropyridine is contacted at elevated temperature with alkali-metal fluoride in a molar ratio alkali metal fluoride: pentachloropyridine in the range 3:1 to 3.9:1 in the solvent, followed by treatment of the resulting 3,5-dichloro-2,4,6-trifluoropyridine with ammonia.

Certain chlorofluoropyridines may be used as intermediates in the preparation of agrochemicals. For this reason polychloropyridines are the preferred feedstocks for the process according to the present invention. Thus 3,5-dichloro-2,4,6-trifluoropyridine can easily be converted into 4-amino-3,5-dichloro-2,6-difluoropyridine, and subsequently into herbicidal 4-amino-3,5-dichloro-6-fluoro-2-(pyridyloxy)acetic acid derivatives, as described in British patent application No. 2,039,473.

The invention will be further understood from the following Examples.

Example I

In two experiments pentachloropyridine (PCPy) was taken up in dry toluene (about 450g toluene per mol PCPY) to which KF was added in a molar ratio of KF : PCPy of 3.5 : 1. To the mixture was added 0.05 mol tetramethylammonium chloride (TMAC) per mol PCPy. The mixture obtained was subjected to azeotropic distillation starting at 121°C whereby about half of the toluene was distilled off, which toluene contained some water (about 1.5%w). To the residue was added an amide, viz. dimethylacetamide (DMAC) or N-methyl-2-pyrrolidone (NMP). The

BK35.010

resulting mixture was heated for a specific period of time, the reaction mixture was then cooled, solid potassium salts were filtered off and the yield of 3,5-dichloro-2,4,6-trifluoro-pyridine (TFPy) in the filtrate was determined. Results and reaction conditions are presented in Table I.

<u>Table 1</u>

| Experiment No. | Amide | | Toluene | Temp. | Reaction | Yield |
|---|---|---|---|---|---|---|
| | name | quantity g/mol KF | quantity g/mol KF | °C | time hrs. | TFPy mol.% |
| 1 | DMAC | 106 | 64 | 135 | 4 | 90 |
| 2 | NMP | 106 | 64 | 142 | 3 | 90 |

<u>Example II</u>

In order to show the importance of the solvent mixture used in the chlorine replacement reactions, experiments were run in which KF, PCPy, the solvent(s) and optionally a phase transfer catalyst (PTC) were placed in a flask, the mixture was heated and stirred, if possible, and solid salts were filtered off. The yields of fluorine-containing derivatives, i.e. trifluoro-(TFPy), difluoro-(DFPy) or monofluoro-(MFPy)pyridine derivatives, were established. Results are shown in Table II.

Table II

| Exp. No. | Solvent | Quantity g/mol KF | PTC | Molar ratio | | | Temp. °C | Reaction time hrs. | Yield | | | Other[2] | Unreacted PCPy mol. % |
|---|---|---|---|---|---|---|---|---|---|---|---|---|---|
| | | | | KF | PCPy | PTC | | | YFPy | DFPy | MFTy mol. % | | |
| 3 | toluene | 197 | TBAC[1] | 3.5 | 1 | 0.02 | 115 | 3 | 0.4 | 3.6 | 24.9 | 4.0 | 67.1 |
| 4 | DMAC | 249 | – | 3.5 | 1 | – | 145 | 1 | 4.0 | 6.0 | 12.0 | 0.0 | 78.0 |
| 5 | NMP | 257 | – | 3.5 | 1 | – | 145 | ·1 | 3.5 | 5.5 | 11.0 | 0.0 | 80.0 |
| 6 | cyano methane | 176 | TBAC[1] | 3.5 | 1 | 0.04 | 83 | 4.5 | 0.8 | 9.8 | 45.9 | 2.5 | 41.0 |
| 7 | dichloro-ethane | 283 | TBAC+[1] TMAC | 3.5 | 1 | 0.02+ 0.02 | 87 | 1 | 0.0 | 0.6 | 15.8 | 0.0 | 83.6 |
| 8 | NMP/ toluene (62/38 %w) | 170 | TMAC | 3.5 | 1 | 0.05 | 142 | 3 | 90.0 | 0.2 | 0 | 9.8 | 0 |

1) TBAC = tetra-n-butylammonium chloride

2) Other products were unidentified heavy compounds.

BK35.010

From the Experiments above it is apparent that only in experiment 8 a satisfactory replacement reaction has taken place, the reaction being carried out in a mixture of NMP and toluene.

Further the reaction mixture in experiments 4 and 5 were unstirrable.

Example III

An experiment was run in which 4-amino-3,5-dichloro-2,4-difluoropyridine was prepared starting from PCPy without isolation of TFPy as intermediate product.

In a vessel 362g toluene, 193g PCPy (0.77 mol) 4.2g TMAC and 160g KF (2.75 mol) were mixed. At 121°C 190g toluene with 3g water was distilled off. Subsequently, to the remaining mixture 285g DMAC was added and the reaction mixture was heated at 135°C for 4 hours. Then solids were filtered off from the reaction mixture (202g) and washed with toluene. The filtrate was purified by flash-distillation yielding 23.5g residue which was discarded. Through the distillate 24g $NH_3$ was bubbled whilst stirring. The temperature was kept at 20°C. The slurry obtained was filtered and the filtered $NH_4F$ was washed with toluene. The filtrate was objected to fractionated distillation yielding toluene and DMAC separately. To the amino product-containing bottoms fraction water was added, the product thereby being precipitated. After filtration, washing and drying 128g amino-product was obtained (0.64mol), representing a yield of 83 mol.% based on PCPy. The product consisted of 94.2% of 4-amino-3,5-dichloro-2,6-difluoropyridine and 5.8% of 2-amino-3,5-dichloro-4,6-difluoropyridine.

K 589 FF

CLAIMS

1. Process for the preparation of a fluorinated pyridine in which a chlorine-containing pyridine derivative is contacted at elevated temperature with an alkali-metal fluoride in a solvent, wherein the solvent comprises a mixture of at least one aprotic amide and at lest one aromatic hydrocarbon.

2. Process according to claim 1, in which the chlorine-containing pyridine derivative is pentachloropyridine.

3. Process according to claim 1 or 2, in which the alkali-metal fluoride is potassium fluoride.

4. Process according to any of claims 1 to 3, in which the molar ratio of alkali-metal fluoride and chlorine atoms to be replaced is in the range 1:1 and 1.3:1.

5. Process according to any one of claims 1 to 4, in which the aprotic amide is N-methylpyrrolidone or N,N-dimethylacetamide.

6. Process according to any one of claims 1 to 5, in which the aromatic hydrocarbon is toluene.

7. Process according to any one of claims 1 to 6, in which the weight ratio of aprotic amide to aromatic hydrocarbon is in the range from 65:35 to 20:80.

8. Process according to any of claims 1 to 7, in which the solvent is present in an amount from 170 to 750g per mol of alkali-metal fluoride.

9. Process according to any of claims 1 to 8, in which the elevated temperature is in the range from 90 to 170°C.

10. Process according to any of claims 1 to 9, in which a phase transfer catalyst is included in the reaction mixture.

11. Process according to claim 10, in which the phase transfer catalyst is tetra$(C_{1-4}$alkyl) ammonium halide.

12. Process according to any of claims 1 to 11 wherein the fluorinated pyridine is difluoropyridine, in which pentachloropyridine is contacted at elevated temperature with alkali-metal fluoride in a molar ratio alkali metal fluoride: pentachloropyridine in the range 3:1 to 3.9:1 in the solvent, followed by treatment of the resulting 3,5-dichloro-2,46-trifluoropyridine with ammonia.